# EUROPEAN PATENT APPLICATION

(11) **EP 4 176 878 A1**
(43) Date of publication of application: **10.05.2023**
(21) Application number: 22200801.3
(22) Date of filing: 09.04.2018
(51) Int. Cl.: A61K 31/454, A61K 31/573, A61K 31/58, A61P 35/00

(54) **COMBINATION THERAPY FOR PROSTATE CANCER**

(30) Priority: 13.04.2017 US 201762485164 P
(62) Divisional of application: 18720895.4
(71) Applicant: JANSSEN Pharmaceutica NV, 2340 Beerse (BE)
(72) Inventor: YU, Margaret K, Los Angeles, 90024 (US); SNYDER, Linda Anne, Spring House, 19477 (US)
(74) Representative: Carpmaels & Ransford LLP

(57) **Abstract**

Provided are methods and compositions, for treating prostate cancer by administering to a patient in need thereof a therapeutically effective amount of a PARP inhibitor, e.g., niraparib; a therapeutically effective amount of a CYP17 inhibitor, e.g., abiraterone acetate, and a therapeutically effective amount of a glucocorticoid, e.g., prednisone.

## Description

### TECHNICAL FIELD

The present disclosure relates to the use of small molecule therapeutic agents for the treatment of a prostate cancer.

### BACKGROUND

Prostate cancer is the most common non-cutaneous malignancy in men and the second leading cause of death in men from cancer in the western world. Prostate cancer results from the uncontrolled growth of abnormal cells in the prostate gland. Once a prostate cancer tumor develops, androgens such as testosterone promote prostate cancer growth. At its early stages, localized prostate cancer is often curable with local therapy including, for example, surgical removal of the prostate gland and radiotherapy. However, when local therapy fails to cure prostate cancer, as it does in up to a third of men, the disease progresses into incurable metastatic disease (i.e., disease in which the cancer has spread from one part of the body to other parts).

For many years, the established standard of care for men with malignant castration-resistant prostate cancer (mCRPC) was docetaxel chemotherapy. More recently, abiraterone acetate (ZYTIGA^{®}) in combination with prednisone has been approved for treating metastatic castrate resistant prostate cancer. Androgen receptor (AR)-targeted agents, such as enzalutamide (XTANDI^{®},) have also entered the market for treating metastatic castrate resistant prostate cancer. Platinum-based chemotherapy has been tested in a number of clinical studies in molecularly unselected prostate cancer patients with limited results and significant toxicities. However, there remains a subset of patients who either do not respond initially, or become refractory (or resistant) to these treatments. No approved therapeutic options are available for such patients.

Niraparib, 2-[4-[(3S)-piperidin-3-yl]phenyl]indazole-7-carboxamide, is an orally available, highly selective poly (adenosine diphosphate [ADP]-ribose) polymerase (PARP) inhibitor, with activity against PARP-1 and PARP-2 deoxyribonucleic acid (DNA)-repair polymerases. Jones P, Wilcoxen K, Rowley M, Toniatti C. Niraparib: A Poly(ADP-ribose) Polymerase (PARP) Inhibitor for the Treatment of Tumors with Defective Homologous Recombination. J Med Chem. 2015 Apr 23;58(8):3302-3314.

PARPs are enzymes responsible for repair of DNA single-strand breaks (SSBs) through a process called base excision repair. PARP inhibition leads to an accumulation of unrepaired SSBs, which result in stalling and collapse of replication forks and, consequently, to double-stranded breaks (DSBs). Normally, DSBs are repaired through homologous recombination (HR). If not repaired, DSBs result in cell death. When tumor cells with DNA-repair defects involving the HR pathway (e.g., Breast Cancer genes [BRCA]-1/2) are treated with a PARP inhibitor, they are unable to efficiently and accurately repair DSBs, which creates a synthetic lethal condition. In men with metastatic castration-resistant prostate cancers (mCRPC), tumors with DNA-repair anomalies account for approximately 20% to 30% of the sporadic cancers.

Recently, the PARP inhibitor, olaparib, was investigated in a Phase 2 study to assess efficacy and safety in patients with mCRPC post-chemotherapy and AR-targeted agents. Genetic sequencing identified homozygous deletions, deleterious mutations, or both in DNA-repair genes, including, but not limited to BRCA-1/2, ATM, Fanconi anemia genes, and CHEK2 in tumor samples. Sixteen of 49 patients had a response (33%; 95% confidence interval [CI]: 20%, 48%). Response was defined as one or more of the following: objective response, circulating tumor cell (CTC) conversion, or prostate specific antigen (PSA) decline ≤50%. Of these 16 patients, 14 (88%) had a response to olaparib and were biomarker-positive for anomalies in DNA-repair genes, including all 7 patients with BRCA-2 loss (4 with bi-allelic somatic loss, and 3 with germline mutations) and 4 of 5 patients with ATM aberrations. Conversely, only 2 of 33 biomarker-negative patient tumors (6%) had a response. Radiographic progression-free survival (rPFS) was significantly longer in the biomarker-positive group than in the biomarker-negative group (median: 9.8 versus 2.7 months, respectively). Overall survival (OS) was also prolonged in the biomarker-positive group versus the biomarker-negative group (median: 13.8 months versus 7.5 months, respectively).

However, a need remains for therapies against a prostate cancer, including hormone sensitive, hormone naive high risk, and castration-resistant prostate cancers.

### SUMMARY

Provided herein are methods for treating a prostate cancer comprising, consisting of and/or consisting essentially of, administering to a patient in need thereof a therapeutically effective amount of a PARP inhibitor, a therapeutically effective amount of a CYP17 inhibitor and a therapeutically effective amount of a glucocorticoid.

Provided herein are methods for treating a prostate cancer comprising, consisting of and/or consisting essentially of administering to a patient in need thereof a therapeutically effective amount of a niraparib, a therapeutically effective amount of a abiraterone acetate and a therapeutically effective amount of prednisone.

Provided herein are methods for treating a prostate cancer comprising, consisting of and/or consisting essentially of administering to a patient in need thereof, a therapeutically effective amount of niraparib, a therapeutically effective amount of abiraterone acetate, and a therapeutically effective amount prednisone.

Also disclosed are pharmaceutical compositions comprising, consisting of and/or consisting essentially of, niraparib, abiraterone acetate, and prednisone in a total amount that is therapeutically effective for the treatment of a prostate cancer in a human patient.

The present disclosure also provides kits comprising consisting of, and/or consisting essentially of, a composition that comprises niraparib; a composition that comprises abiraterone acetate; and a composition that comprises prednisone; and, an instruction print for administering the compositions to a human patient having a prostate cancer.

### DESCRIPTION OF THE FIGURES

Figure 1 illustrates the effect of niraparib and/or abiraterone on tumor volume in castrated male mice bearing VCaP tumors.
Figure 2 illustrates the effect of niraparib and/or abiraterone on survival in castrated male mice bearing VCaP tumors.

### DETAILED DESCRIPTION OF ILLUSTRATIVE EMBODIMENTS

The present inventions may be understood more readily by reference to the following detailed description, taken in connection with the accompanying examples, which form a part of this disclosure. It is to be understood that these inventions are not limited to the specific products, methods, conditions or parameters described and/or shown herein, and that the terminology used herein is for the purpose of describing particular embodiments by way of example only and is not intended to be limiting of the claimed inventions.

The entire disclosures of each patent, patent application, and publication cited or described in this document are hereby incorporated herein by reference.

As employed above and throughout the disclosure, the following terms and abbreviations, unless otherwise indicated, shall be understood to have the following meanings.

In the present disclosure the singular forms "a,", "an," and "the" include the plural reference, and reference to a particular numerical value includes at least that particular value, unless the context clearly indicates otherwise. Thus, for example, a reference to "an ingredient" is a reference to one or more of such ingredients and equivalents thereof known to those skilled in the art, and so forth. Furthermore, when indicating that a certain element "may be" X, Y, or Z, it is not intended by such usage to exclude in all instances other choices for the element.

When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" refers to a value of 7.2 to 8.8, inclusive; as another example, the phrase "about 8%" refers to a value of 7.2% to 8.8%, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", and the like. In addition, when a list of alternatives is positively provided, such a listing can also include embodiments where any of the alternatives may be excluded. For example, when a range of "1 to 5" is described, such a description can support situations whereby any of 1, 2, 3, 4, or 5 are excluded; thus, a recitation of "1 to 5" may support "1 and 3-5, but not 2", or simply "wherein 2 is not included."

### DEFINITIONS

As used herein, and unless otherwise defined, the terms "treat," "treating" and "treatment" include the eradication, removal, modification, management or control of a tumor or primary, regional, or metastatic prostate cancer cells or tissue and the minimization or delay of the spread of prostate cancer.

As used herein, and unless otherwise defined, the phrase "therapeutically effective amount" means an amount of the therapeutic agent effective for treating a prostate cancer.

As used herein, and unless otherwise defined, the phrase "safe therapeutic" means an amount of the therapeutic agent effective for treating a prostate cancer.

The present disclosure relates, *inter alia,* to methods for treating prostate cancer comprising administering to a patient in need thereof a therapeutically effective amount of niraparib, abiraterone acetate, and prednisone. As noted above, current therapeutic options for men with metastatic castration-resistant prostate cancer (mCRPC) that improve survival and limit progression include taxane-based chemotherapy, and androgen receptor-targeted agents such as apalutamide and enzalutamide (XTANDI^{®}). However, there remains a subset of patients who either do not respond initially or become refractory to these treatments. No approved therapeutic options are available for such patients.

In response to genotoxic insult, poly (adenosine diphosphate [ADP]-ribose) polymerases (PARPs) recruit proteins that promote DNA repair. Recent studies have observed dual roles for PARP in supporting androgen receptor activity, in addition to the facilitation of DNA repair. Namely, models of CRPC showed marked up-regulation of PARP activity. Accordingly, PARP inhibition in preclinical models enhanced the antitumor effects of castration and delayed onset to castration resistance. Moreover, primary human prostate tumor *ex vivo* cultures revealed a significant antitumor response to PARP inhibition, which was correlated with diminished androgen receptor activity. Similarly, in preclinical models, a marked decrease in the survival of cells co-treated with the anti-androgen, bicalutamide, and an ATM inhibitor was observed in colony formation assays when compared with those treated with either bicalutamide or an ATM inhibitor alone. Given the ability of androgen receptor signaling to remain after castration therapy in CRPC, the present inventors identified a major role for the potential synergy of PARP inhibition and androgen receptor antagonism.

Niraparib, illustrated below, is an orally available, highly selective poly (adenosine diphosphate [ADP]-ribose) polymerase (PARP) inhibitor, with potent activity against PARP-1 and PARP-2 deoxyribonucleic acid (DNA)-repair polymerases.

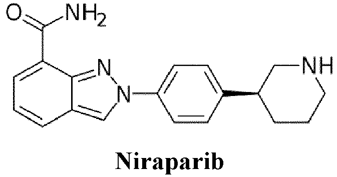

The preparation of niraparib, 2-[4-[(3 S)-piperidin-3-yl]phenyl]indazole-7-carboxamide, is described in U.S. Patent Nos. 8,071,623 and 8,436,185, both of which are incorporated herein by reference. Niraparib is being investigated for the treatment of ovarian, breast, and prostate tumors in patients with DNA-repair anomalies.

In cancer cell lines that have been silenced for BRCA-1 or BRCA-2 genes, or that have BRCA-1 and BRCA-2 mutations, niraparib demonstrates antiproliferative activity due to cell cycle arrest, followed by apoptosis. Further, *in vivo* studies in mice showed that niraparib has antitumor activity against BRCA-1-deficient breast, BRCA-2-deficient pancreatic and ovarian cancers.

Niraparib is not a potent inhibitor or inducer of cytochrome P450 (CYP) enzymes; therefore, it is considered to have low potential to cause drug-drug interactions (DDIs). However, preclinical data show that niraparib has a moderate potential to induce CYP1A2 enzymes. Niraparib is a low clearance drug, and limited metabolism with involvement of multiple metabolic enzymes (both CYPs and non-CYPs) was observed *in vitro.* Therefore, no major changes in niraparib pharmacokinetics (PK) are expected when co-administered with modulators of CYP enzymes. Niraparib is a P-glycoprotein substrate. In animal studies, distribution of niraparib to the central nervous system (CNS) was observed; however, niraparib had no effect on neurological function in a CNS safety study using conscious mice. QTc interval was unaffected in anesthetized dogs; however, mean arterial pressure, heart rate, and QRS cardiac interval were increased. In repeat-dose toxicity studies in rats and dogs, hematologic toxicities were observed but resolved by the end of the recovery period (typically 15 to 28 days).

While niraparib is preferred any PARP inhibitor, such as talazoparib, rucaparib, olaparib, iniparib, talazoparib, and veliparib, is also contemplated to be useful in the present invention. Abiraterone acetate (available under the trade name ZYTIGA^{®}), illustrated below, is a 17a-hydroxylase/C17,20-lyase (CYP17) inhibitor that blocks androgen biosynthesis in the testes, adrenal gland, and prostate tumor.

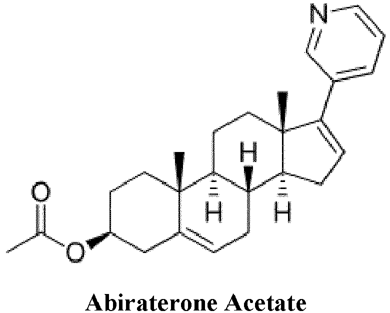

Abiraterone, the active metabolite of abiraterone acetate, inhibits CYP17A1, which manifests as two enzymes, 17α-hydroxylase (IC₅₀ = 2.5 nM) and 17,20-lyase (IC50 = 15 nM) (six-fold more selective for inhibition of 17α-hydroxylase over 17,20-lyase) that are expressed in testicular, adrenal, and prostatic tumor tissues. CYP 17 catalyzes two sequential reactions: (1) the conversion of pregnenolone and progesterone to their 17α-hydroxy derivatives by its 17α-hydroxylase activity, and (2) the subsequent formation of dehydroepiandrosterone (DHEA) and androstenedione, respectively, by its 17,20-lyase activity. DHEA and androstenedione are androgens and precursors of testosterone. Inhibition of CYP17 activity by abiraterone thus decreases circulating levels of androgens such as DHEA, testosterone, and dihydrotestosterone (DHT).

Administration of abiraterone acetate has the capacity to lower circulating testosterone levels to less than 1 ng/dL (i.e., undetectable), and these concentrations are much lower than those achieved by castration (20 ng/dL). The addition of the effects of administration of abiraterone acetate to castration was found to reduce levels of DHT by 85%, DHEA by 97-98%, and androstenedione by 77-78% relative to castration alone.

Niraparib and abiraterone acetate may also be administered or combined with steroids, such as corticosteroids or glucocorticoids. Niraparib and abiraterone acetate and the steroid may be administered in the same or in different compositions. Non-limiting examples of suitable steroids include hydrocortisone, prednisone, prednisolone, methyl-prednisolone or dexamethasone. The amount of the steroid administered is an amount that is sufficient to treat the prostate cancer whether administered alone or in combination with niraparib and abiraterone acetate.

In one embodiment, provided herein are methods and compositions comprising niraparib, abiraterone acetate and a steroid particularly a corticosteroid, or more particularly a glucocorticoid. Steroids within the scope of the disclosure include, but are not limited to, (1) hydrocortisone (cortisol; cyprionate (e.g., CORTEF), oral; sodium phosphate injection (HYDROCORTONE PHOSPHATE); sodium succinate (e.g., A-HYDROCORT, Solu-CORTEF); cortisone acetate oral or injection forms, etc.), (2) dexamethasone (e.g., Decadron, oral; Decadron-LA injection, etc.), (3) prednisolone (e.g., Delta-CORTEF, prednisolone acetate (ECONOPRED), prednisolone sodium phosphate (HYDELTRASOL), prednisolone tebutate (HYDELTRA-TBA, etc.)), or (4) prednisone DELTASONE, etc.) and combinations thereof. See, e.g., GOODMAN & GII,MAN'S THE PHARMACOLOGICAL BASIS OF THERAPEUTICS, 10TH EDITION 2001.

In a specific embodiment, single unit solid oral dosage forms which comprise an amount from about 50 mg to about 300 mg of abiraterone acetate and an amount from about 0.5 mg to about 3.0 mg of a steroid, e.g., glucocorticoid in a single composition, optionally with excipients, carriers, diluents, etc. is contemplated. For instance, the single unit dosage form can comprise about 250 mg of abiraterone acetate and about 1.0 mg, 1.25 mg, 1.5 mg, or 2.0 mg of a steroid, such as but not limited to corticosteroids or glucocorticoids.

Abiraterone acetate is indicated for use in combination with prednisone as a treatment for metastatic castration-resistant prostate cancer.

In accordance with the present methods, the patient to whom the niraparib, the abiraterone acetate, and prednisone are administered may have hormone-sensitive prostate cancer, hormone naive localized high-risk prostate cancer, or castration-resistant prostate cancer. Most, but not all, prostate cancers are adenocarcinomas, and the patient may have adenocarcinoma or sarcoma-based prostate cancer. In any of these instances, the prostate cancer may be metastatic.

The patient may have undergone one or more other types of treatment for the prostate cancer prior to the first dose of the niraparib, abiraterone acetate, and prednisone. For example, the patient may have undergone taxane-based chemotherapy prior to administering a first dose of the niraparib, abiraterone acetate, and prednisone. Additionally or alternatively, the patient may have undergone at least one line of androgen receptor-targeted therapy prior to administering a first dose of the niraparib, abiraterone acetate, and prednisone. The period of time between the end of the other treatment and the administration of niraparib, abiraterone acetate, and prednisone in accordance with the present invention may be years, months, weeks, days, a single day, or less than 24 hours.

The niraparib, abiraterone acetate, and/or prednisone may be administered to the patient in a single composition, such as a single pill (e.g., a tablet or a capsule), bolus injection, or the like. Alternatively, the niraparib, abiraterone acetate, and prednisone may be administered to the patient separately, i.e., such that the niraparib, abiraterone acetate, and prednisone are physically separate from one another prior to administration. For example, the niraparib abiraterone acetate may be administered in a first dosage form, while the prednisone is administered to the patient in a second, separate dosage form, or the niraparib, abiraterone acetate, and prednisone may be administered in separate dosage forms. When the niraparib, abiraterone acetate, and prednisone are administered to the patient separately, the period of time between the respective administrations is preferably minimal (e.g., less than about 6 hours) or effectively zero (e.g., such as when the patient ingests two separate dosage forms simultaneously, one of the dosage forms containing the niraparib, and the other respective dosage forms containing abiraterone acetate and prednisone), but may be about one minute, about 2 minutes, about 3 minutes, about 5 minutes, about 7 minutes, about 10 minutes, about 15 minutes, about 20 minutes about 25 minutes, about 30 minutes, about 40 minutes, about 45 minutes, about 50 minutes, about 60 minutes, about 70 minutes, about 80 minutes, about 90 minutes, about 2 hours, about 3 hours, about 4 hours, about 5 hours, about 6 hours, about 7 hours, about 8 hours, about 9 hours, about 10 hours, about 12 hours, about 13 hours, about 14 hours, or about 15 hours. Pharmaceutical compositions comprising niraparib, abiraterone acetate, and prednisone, or all three are described more fully *infra.*

The administration of the niraparib, abiraterone acetate, and prednisone may be on a once daily basis. In other words, the niraparib may be administered on a once daily basis, the abiraterone acetate may be administered on a once daily basis, and the prednisone may be administered on a once daily basis. In some instances, the once daily administration includes administering a single composition comprising the niraparib, abiraterone acetate, and prednisone to the patient one time per day. In other embodiments, the once daily administration includes administering a single composition comprising the niraparib to the patient once time per day, administering a second composition comprising the abiraterone acetate to the patient one time per day, and administering a third composition comprising the prednisone to the patient one time per day. The once daily administration may alternatively include administering one or more compositions collectively making up the once daily dosage of the niraparib to the patient during a single day period, administering one or more compositions collectively making up the once daily dosage of the abiraterone acetate to the patient during a single day period, and administering two or more compositions making up a two- or more times-daily dosage of the prednisone. For example, the niraparib may be administered to the patient in a single composition to the patient one time per day, while the abiraterone acetate is administered to the patient in the form of two or more separate dosage forms one time per day, and the prednisone is administered to the patient in the form of two or more separate dosage forms one time each per day. Any dosage regimen that is embraced by the preceding description is contemplated.

The amount of niraparib that is administered to the patient may be about 30 to about 400 mg/day, about 50 to about 350 mg/day, about 75 to about 325 mg/day, about 100 to about 300 mg/day, about 100 to about 275 mg/day, about 125 to about 250 mg/day, about 150 to about 225 mg/day, about 175 to about 225 mg/day, or about 190 to about 210 mg/day, or, about 30, about 40, about 50, about 60, about 70, about 80, about 90, about 100, about 110, about 120, about 130, about 140, about 150, about 160, about 170, about 180, about 190, about 200, about 210, about 220, about 230, about 240, about 250, about 260, about 270, about 280, about 290, about 300, about 310, about 320, about 330, about 340, or about 350 mg/day.

The amount of abiraterone acetate that is administered to the patient may be about 500 to about 1500 mg/day, about 600 to about 1300 mg/day, about 700 to about 1200 mg/day, about 800 to about 1200 mg/day, about 900 to about 1100 mg/day, about 950 to about 1050 mg/day, or may be about 500, about 600, about 700, about 750, about 800, about 850, about 875, about 900, about 925, about 950, about 1000, about 1025, about 1050, about 1075, about 1100, or about 1125 mg/day.

The amount of prednisone that is administered to the patient may be about 1 to about 25 mg/day, about 2 to about 23 mg/day, about 3 to about 20 mg/day, about 4 to about 18 mg/day, about 5 to about 15 mg/day, about 6 to about 12 mg/day, about 7 to about 11 mg/day, about 8 to about 11 mg/day, about 9 to about 11 mg/day, or may be about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, or about 25 mg/day.

The present methods may include administering the niraparib, the abiraterone acetate, and the prednisone to the patient over multiple days, weeks, months or years. Preferably, the administration of the niraparib and the abiraterone acetate, occurs on a once daily basis, and the administration of the prednisone occurs on a twice daily basis. The amount of the niraparib, the abiraterone acetate, and the prednisone may be constant over time (i.e., from day to day), or may be increased or decreased over time. For example, the amount of niraparib, the abiraterone acetate, and the prednisone, or two or all three of these, that is administered per day may be increased or decreased after one day of administration, after a few days of administration, after a week of administration, after, and the new dosage amount may be maintained for any desired period of time, **e.g.**, days, weeks, or months, or may subsequently be increased or decreased after the desired interval. In this manner, the present methods may include increasing the dosing of niraparib (**e.g.**., the amount of the niraparib that is administered on a once daily basis) at least once over time. The present methods may also or alternatively include increasing the dosing of abiraterone acetate (e.g.., the amount of the abiraterone acetate that is administered on a once daily basis) at least once over time. The present methods may also or alternatively include increasing the dosing of prednisone (*e.g.*., the total amount of the prednisone that is administered on a daily basis) at least once over time. The amount of increase or decrease may be expressed in terms of a percentage, and under such circumstances the amount of a single episode of increase or decrease may be about 5%, about 10%, about 15%, about 20%, about 25%, about 30%, about 35%, about 40%, about 45%, about 50%, about 55%, about 60%, about 65%, about 70%, about 80%, about 85%, about 90%, about 95%, about 100%, or by greater than about 100%.

Described herein are methods for treating a cancer in which a therapeutically effective amount of PARP inhibitor, such as niraparib, a 17α-hydroxylase/C17,20-lyase inhibitor, such as abiraterone acetate (i.e,. 3β-acetoxy-17-(3-pyridyl)androsta-5,16-diene), and a glucocorticoid, such as prednisone, prednisolone, hydrocortisone, methylpresnisolone, and dextamethasone, are administered to a patient, e.g., a patient in need thereof, in combination with a therapeutically effective amount of at least one additional therapeutic agent including, but not limited to, an anti-cancer agent or steroid. Such methods can also provide an effective treatment for individuals with a refractory cancer, including individuals who are currently undergoing a cancer treatment. Therefore, in certain embodiments, the method is directed to treating a resistant prostate cancer in a patient, in which a therapeutically effective amount of 17α-hydroxylase/C17,20-lyase inhibitor is administered to a patient currently receiving an anti-cancer agent.

In such compositions, the niraparib may be present in an amount that is therapeutically effective by itself, the abiraterone acetate may be present in an amount that is therapeutically effective by itself, the prednisone may be present in an amount that is therapeutically effective by itself, or two or more of these conditions may apply. In other embodiments, the total amount of the niraparib, the abiraterone acetate, and the prednisone when considered together may represent a therapeutically effective amount, *i.e.,* the amount of niraparib would not be therapeutically effective by itself, the amount of abiraterone acetate would not be therapeutically effective by itself, and the amount of prednisone would not be therapeutically effective by itself. The "therapeutically effective amount" for purposes of the present compositions is defined as described above in connection with the instant methods.

The compositions containing the active ingredients may be in a form suitable for oral use, for example, as tablets, troches, lozenges, aqueous or oily suspensions, dispersible powders or granules, emulsions, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredients in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium croscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water-soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredients are mixed with an inert solid diluent, for example, calcium carbonate, calcium phosphate or kaolin, or as soft gelatin capsules wherein the active ingredients are mixed with water soluble carrier such as polyethyleneglycol or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanal, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredients in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredients in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsion. The oily phase may be a vegetable oil, for example olive oil or arachisoil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives, and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solution. Among the acceptable vehicles and solvents that may be employed are, for example, water, Ringer's solution, and isotonic sodium chloride solution.

The sterile injectable preparation may also be a sterile injectable oil-in-water microemulsion where the active ingredients are dissolved in the oily phase. For example, the active ingredients may be first dissolved in a mixture of soybean oil and lecithin. The oil solution then introduced into a water and glycerol mixture and processed to form a microemulsion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS^{™} model 5400 intravenous pump.

The pharmaceutical compositions may be in the form of a sterile injectable aqueous or oleaginous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of inj ectables.

The compositions comprising niraparib, abiraterone acetate, and prednisone may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, and the like, containing the instant compounds are employed. For purposes of the present disclosure, topical application shall include mouth washes and gargles.

The compositions comprising niraparib, abiraterone acetate, and prednisone can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

When the compositions comprising niraparib, abiraterone acetate, and prednisone are administered to a patient, the selected dosage level for each drug will depend on a variety of factors including, but not limited to, the activity of the particular compound, the severity of the individual's symptoms, the route of administration, the time of administration, the rate of excretion of the compound, the duration of the treatment, other drugs, compounds, and/or materials used in combination, and the age, sex, weight, condition, general health, and prior medical history of the patient. The amount of niraparib, the amount of abiraterone acetate, and the amount of prednisone, and the route of administration will ultimately be at the discretion of the physician, although generally the dosage will be to achieve local concentrations at the site of action which achieve the desired effect without causing substantial harmful or deleterious side-effects.

The compositions may comprise, for example, about 100 to about 350 mg of the niraparib, about 100 to about 1500 mg of the abiraterone acetate, and about 2 to about 15 mg of the prednisone.

For example, the instant compositions may include niraparib in an amount of, for example, 100 to about 350 mg, about 100 to about 340 mg, about 125 to about 340 mg, about 150 to about 325 mg, about 175 to about 325 mg, about 200 to about 300 mg, or may be about 100, about 110, about 120, about 130, about 140, about 150, about 160, about 170, about 180, about 190, about 200, about 210, about 220, about 230, about 240, about 250, about 260, about 270, about 280, about 290, about 300, about 310, about 320, about 330, about 340, or about 350 mg.

The instant compositions may also include abiraterone acetate in an amount of, for example, about 100 to about 1500 mg, about 125 to about 1400 mg, about 150 to about 1300 mg, about 175 to about 1200 mg, about 200 to about 1175 mg, about 225 to about 1150 mg, about 250 to about 1100 mg, about 250 to about 1075 mg, about 250 to about 1050 mg, about 250 to about 1000 mg, about 300 to about 950 mg, about 350 to about 900 mg, about 400 to about 850 mg, about 450 to about 800 mg, or about 500 to about 700 mg, or may be about 100, about 150, about 175, about 200, about 225, about 250, about 275, about 300, about 325, about 350, about 375, about 400, about 450, about 500, about 550, about 600, about 650, about 700, about 750, about 800, about 850, about 900, about 950, about 1000, about 1050, about 1100, about 1150, about 1200, about 1250, about 1300, about 1350, about 1400, about 1450, or about 1500 mg.

The present compositions may also include prednisone in an amount of, for example, about 2 about 15 mg, about 2 to about 14, about 3 to about 13, about 4 to about 12, about 5 to about 11, about 5 to about 10, about 6 to about 11, about 7 to about 11, about 8 to about 11, about 9 to about 11, or may be about 2, about 3, about 4, about 5, about 6, about 7, about 8, about 9, about 10, about 11, about 12, about 13, about 14, or about 15 mg.

Also disclosed herein are kits including a composition that comprises niraparib, a composition that comprises abiraterone acetate, a composition that comprises prednisone, and, an instruction print for administering the compositions to a human patient having prostate cancer. The instruction print may provide instructions for administering the respective compositions once daily, twice daily, or multiple-times daily. For example, the instruction print may provide instructions for administering the composition comprising niraparib and the composition comprising abiraterone acetate to a human patient having prostate cancer on a once daily basis, and for administering the composition comprising prednisone to the human patient on a twice daily basis.

In the instant kits, the composition that comprises niraparib, the composition that comprises abiraterone acetate, and the composition that comprises prednisone may represent separate compositions, or two or more of the composition that comprises niraparib, the composition that comprises abiraterone acetate, and the composition that comprises prednisone may be one and the same. In other words, in the latter condition, the instant kits comprise a composition according to the present disclosure that comprises two or all of niraparib, abiraterone acetate, and prednisone as described *supra.* Whether the composition that comprises niraparib, the composition that comprises abiraterone acetate, and the composition that comprises prednisone respectively represent separate compositions or not, the respective compositions may have of the characteristics described above for the inventive pharmaceutical compositions. Thus, the composition or compositions of the instant kits may be configured for any desired route of administration and contain any excipients, additives, and amounts of respective drugs in accordance with the preceding description, e.g., the respective compositions may comprise, for example, about 100 to about 350 mg of the niraparib, about 100 to about 1500 mg of the abiraterone acetate, and about 2 to about 15 mg of the prednisone (or any combination of these) in accordance with the preceding description concerning the instant compositions.

In some instances, the composition that includes abiraterone acetate is a dosage form that is configured for oral administration to a patient *(i.e.,* an oral dosage form), the composition that comprises prednisone is a dosage form that is configured for oral administration to a patient, and the composition that comprises niraparib is a dosage form that is configured for oral administration to a patient, and the kit includes two to five of the dosage forms comprising abiraterone acetate for every one dosage form including niraparib, and one to three of the dosage forms including prednisone for everyone dosage form comprising niraparib. Accordingly, the intended administration regimen for such kits would be to administer one of the dosage forms including niraparib for every two to five of the dosage forms comprising abiraterone acetate and for every one to three of the dosage forms comprising prednisone that are administered to a patient. For example, the once-daily administration associated with such kits may comprise administering a single dosage form, such as a capsule, comprising niraparib, two to five of the dosage forms comprising abiraterone acetate, and one to three of the dosage forms comprising prednisone. In such instances, the respective dosage forms comprising abiraterone acetate and prednisone may individually contain less abiraterone acetate and prednisone than would provide a therapeutically effective dose, but, because they are intended to be taken in groups of two to five or one to three, respectively, the combination of the two to five or one to three dosage forms would represent a therapeutically effective amount of abiraterone acetate and prednisone, respectively. In one embodiment, the administration associated with the instant kits comprises administering a single dosage form comprising niraparib, four dosage forms comprising abiraterone acetate, and two dosage forms comprising prednisone.

In certain instances, one or more of the niraparib, abiraterone acetate, and prednisone are administered in a multiple-times-daily regimen, and one or both of the other of the niraparib, abiraterone acetate, and prednisone are administered on a once daily basis. For example, the niraparib and abiraterone acetate may be administered on a once daily basis, while the prednisone is administered on a twice- or three-times daily basis. One embodiment involves administering the niraparib and abiraterone acetate on a once daily basis, and administering the prednisone on a twice daily basis. In a particular embodiment, the administration associated with the instant kits comprises administering a single dosage form comprising niraparib, four dosage forms comprising abiraterone acetate, and two dosage forms comprising prednisone, wherein the niraparib and abiraterone acetate are administered on a once daily basis *(i.e.,* each of the separate dosage forms are administered together in a single instance), and the prednisone is administered on a twice daily basis (i.e., one of the dosage forms including prednisone is administered at a certain point in time during a particular day, and the second of the dosage forms including prednisone is administered at a later point in time during a particular day, such as four hours, five hours, six hours, seven hours, eight hours, nine hours, 10 hours, 11 hours, or 12 hours later).

Depending on which of the preceding administration regimens, or another regimen, applies, the instruction print will provide appropriate guidance for providing the once daily administration of the composition or compositions that are included in the kit.

The present invention is further defined in the following examples. It should be understood that these examples, while indicating preferred embodiments of the invention, are given by way of illustration only, and should not be construed as limiting the appended claims. From the above discussion and these examples, one skilled in the art can ascertain the essential characteristics of this invention, and without departing from the spirit and scope thereof, can make various changes and modifications of the invention to adapt it to various usages and conditions.

### EXAMPLES

### Example 1- Combination Therapy with Patient Evaluation

Niraparib is provided as 200 mg capsules or tablets for once daily oral administration to a patient, abiraterone acetate is provided as capsules or tablets (4 × 250 mg, total dose 1000 mg) for once daily oral administration, and prednisone is provided as tablets or capsules (2 × 5 mg) for twice daily oral administration (one 5 mg tablet each of two times daily). Dosing of niraparib is initiated at a starting dose of 200 mg once daily, 67% of the current clinical dose for niraparib monotherapy. The dose of abiraterone acetate, 1000 mg once daily, remains unchanged throughout the treatment period, and the dose of prednisone, 5 mg twice daily, remains unchanged as well during the treatment period. All drugs are administered together from Cycle 1 Day 1 onwards. The drugs must be swallowed whole. Patients take their dose in the morning (with or without food), except on days when pharmacokinetic (PK) sampling occurs. Water is permitted. A snack (e.g., crackers, cheese, and juice) is given after the 2 hour PK blood sample is collected, a standard lunch (e.g., turkey submarine sandwich with cheese, lettuce, tomato, and spread plus one cup of tea or coffee) is provided after the 4 hour PK blood sample is collected, and a standard dinner (e.g., chicken, broccoli, and rice plus 1 cup of tea or coffee) is provided after the 8 hour PK blood sample is collected.

*Pharmacokinetic evaluation.* Blood samples are collected over a 24-hour dosing interval on Cycle 1 Day 28 as per the Time and Events Schedule for PK and Pharmacodynamics Assessments. Plasma levels of niraparib and its major metabolite (M1), as well as abiraterone acetate and its active metabolite, abiraterone, are measured, along with the plasma levels of prednisone. The following steady-state key PK parameters of niraparib, abiraterone acetate, and prednisone are calculated.

*Antitumor activity evaluation.* The antitumor activity evaluations include the following:

| | |
|---|---|
| • tumor measurements: Chest, abdomen, and pelvis CT or MRI scans and whole body bone scans (^{99m}Tc), | |
| • serum prostate specific antigen (PSA) | • survival status |
| • CTC | • SSEs |

Prostate specific antigen (PSA) evaluations are conducted, applying Prostate Cancer Working Group 3 (PCWG3) recommendations. During the treatment phase, radionuclide bone scans or CT/MRI imaging assessments are optionally performed at the discretion of the investigator in accordance with institutional guidelines, in case of observation of PSA progression or suspicion of clinical progression.

All enrolled subjects will be analyzed. Evaluations for antitumor activity will be conducted. Assessments. PSA evaluations and bone progression assessments will be based on PCWG3 recommendations, soft tissue (visceral and/or nodal disease) evaluations will be based on Response Evaluation Criteria in Solid Tumors (RECIST) Version 1.1.

Antitumor activity will be analyzed as follows:
- PSA decline at 12 weeks: PSA percent change from baseline (rise or fall) at 12 weeks using a waterfall plot.
- Maximum PSA decline: the maximum change (rise or fall) at any time using a waterfall plot.
- CTC response defined as CTC=0 per 7.5 mL blood at 8 weeks post-baseline.
- Objective response rate (ORR) of soft tissue (visceral and/or nodal disease) as defined by RECIST Version 1.1 with no evidence of bone progression according to the PCWG3 criteria.

The analysis for response rates will be tabulated and its 2-sided 95% exact confidence interval will also be presented.

Antitumor evaluations will be conducted as specified in Time and Events Schedules 1 and 2. Unscheduled assessments should be considered if clinically indicated, and results collected in the eCRF. The antitumor activity evaluations include the following:
Tumor measurements: Chest, abdomen, and pelvis CT or MRI scans and whole body bone scans (^{99m}Tc). The same imaging modality should be used throughout the evaluation of an individual subject. Imaging will be assessed by the investigator. After first documentation of response or progression, repeat imaging is required approximately 4 weeks later for confirmation.

| | |
|---|---|
| • serum PSA | • survival status |
| • CTC | • symptomatic skeletal events (SSEs) |

Evaluation of treatment response by PSA, imaging, and CTCs will be performed according to PCWG3 criteria. SSEs should be assessed according to PCWG3 criteria. Progression by tumor assessments and PSA should be evaluated according to PCWG3.

*Pharmacodynamics.* Blood samples for isolation of peripheral blood mononuclear cells (PBMCs) are collected. Lysates of peripheral blood mononuclear cells (PBMCs) are used to quantify polyADP-ribose in PBMCs by chemiluminescent enzyme-linked immunosorbent assay.

*Biomarker evaluations.* All patients have tumor tissue (archival or recently collected) and blood samples collected during the Screening Phase for determination of biomarker analysis; results do not determine eligibility.

### Biomarker-positive sample for DNA-repair anomalies

To evaluate if patients are biomarker-positive, a blood-based assay may become available during the study that will provide a more rapid method than tissue-based analysis for determining biomarker-positive status, while being more convenient for the patients. Prior to the blood-based assay becoming available, tumor tissue (either archival or recently collected) will require analysis. To ensure that all patients, regardless of when they enter the study, have the same biomarker data available for analysis (i.e., for concordance and bridging studies), both tumor tissue and blood samples will be collected from all patients who sign the prescreening informed consent form (ICF). The process for determining biomarker-positivity will be different for patients who enter the Prescreening Phase before the blood-based assay is available, compared with those patients who enter after the blood-based assay is available. However, the status of biomarker-positivity in both tumor tissue and blood will be assessed for all patients.

To be eligible for the study, patients must be confirmed biomarker-positive by tumor tissue (either archival or recently collected), or blood testing when available. The biomarkers of interest for this study and the biomarker-positivity criteria are listed in Table 1. Analyses will be performed to define a proxy for bi-allelic loss (e.g., mutation co-expression frequency with copy number loss) and these proxies may be used to determine biomarker-positivity as that information becomes available.

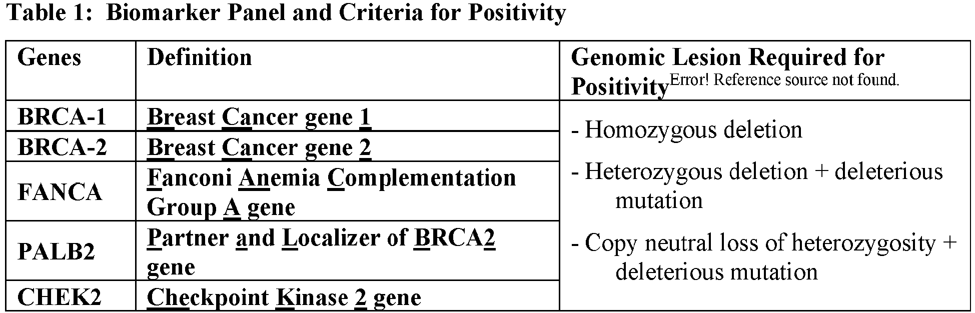

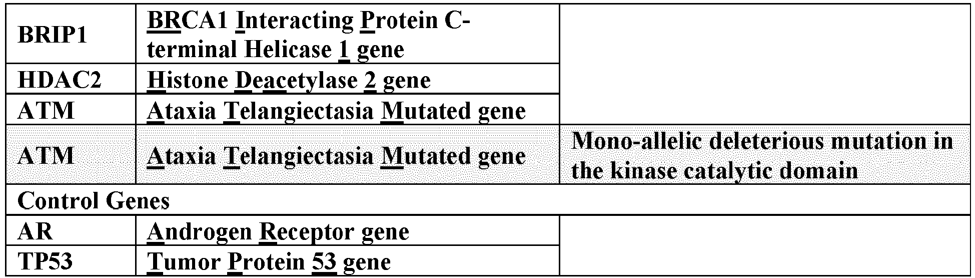

Mono-allelic loss in all genes will be acceptable for entry into the study until the existing algorithm for bi-allelic loss is validated.

### Circulating Tumor Cells

Blood samples will be collected in a Cellsave tube at timepoints specified in the Time and Events Schedule. CTC enumeration will be evaluated at the central laboratory, to assess response to study drug.

### Whole Blood for RNA

Whole blood samples will be collected in a Paxgene tube. Multiple ribonucleic acid (RNA) transcripts found in prostate tumors are detectable in the RNA and analysis of these samples will allow evaluation of potential mechanisms of resistance that may emerge with niraparib.

### Circulating Tumor DNA

Plasma samples collected during the course of treatment will be used to screen for changes in the levels or types of DNA-repair anomalies observed over time by circulating tumor DNA (ctDNA), and to monitor for potential markers of resistance to niraparib.

All subjects will have tumor tissue (archival or recently collected) and blood samples collected during the Screening Phase for determination of biomarker analysis; results do not determine eligibility. Evaluation of the association between biomarkers and antitumor activity may be conducted. Concordance of genomic alterations between tumor DNA and circulating tumor DNA may also be assessed. Circulating tumor cells (CTCs) will be evaluated for response to study drugs.

*Safety evaluation.* Safety assessments are based on medical review of adverse event reports and the results of vital sign measurements, electrocardiograms (12-lead), physical examinations, clinical laboratory tests, and Eastern Cooperative Oncology Group Performance Status.

*Pharmacokinetics and Pharmacokinetics*/*Pharmacodynamics Analyses.* Individual and mean plasma concentration-time data are summarized with descriptive statistics and concentration-time profiles of niraparib and its major metabolite (M1), as well as abiraterone acetate and its active metabolite (abiraterone), will be plotted. PK parameters are summarized with descriptive statistics. Relationships among niraparib, abiraterone acetate, prednisone, and pharmacodynamics or clinical activity or safety endpoints are optionally evaluated.

*Antitumor Activity Analyses.* Patient is analyzed for antitumor activity. PSA evaluations are based on PCWG3 recommendations and as follows: PSA response rate: a ≥50% decline from baseline during the study and are confirmed by a second measurement 3 to 4 weeks later; PSA response at 12 weeks: percent change from baseline (rise or fall) at 12 weeks using a waterfall plot; maximum PSA decline of ≥50%: the maximum change (rise or fall) at any time using a waterfall plot.

*Safety Analyses.* Safety parameters evaluated are the incidence, intensity, and type of AEs, vital signs measurements, ECGs, physical examination (abnormalities will be recorded as AEs), ECOG PS, and clinical laboratory results.

### Example 2 - Efficacy of niraparib plus abiraterone in a human prostate tumor model (VCaP) engrafted in castrated male mice

Purpose: This study evaluated the efficacy of niraparib combined with abiraterone in castrated male mice bearing human VCaP prostate tumors. The readouts were tumor volume and survival.

### Cell Culture

The human VCaP prostate tumor line was derived from a vertebral metastasis of a patient with castrate-resistant prostate cancer. The VCaP tumor cells carry a TMPRSS2-ERG fusion, and express the androgen receptor. The VCaP tumor cell line was maintained in DMEM medium supplemented with 10% fetal bovine serum at 37C in an atmosphere of 5% CO2 in air. The tumor cells were subcultured twice weekly by trypsin-EDTA treatment. The cells were harvested when in exponential growth phase for tumor injection.

### Tumor Cell Injection and Study Design

Each mouse was injected subcutaneously at the right flank with VCaP tumor cells (1 x 10⁷) in 0.1 ml of PBS with Matrigel (1:1) for tumor development. The date of tumor cell inoculation was denoted as day 0. Mice were distributed to treatment groups in order to ensure mean tumor size of approximately 200 mm³, and at that time castration was performed on all mice. Treatment was initiated 1 day after castration as outlined in Table 2, which shows the test article administration and the animal numbers in each group. The date of grouping prior to treatment initiation was denoted as day 0 of post grouping (PG-D0). Dosing continued for 35 days (day 57) and mice were monitored for tumor volume during dosing and until each mouse reached study endpoint. Mice were euthanized when tumor volume reached 1500 mm³.

Tumor volumes were measured twice per week in two dimensions using a caliper, and the volume expressed in mm³ using the formula: V = 0.5 *a* x *b*² where *a* and *b* are the length and width of the tumor, respectively.

**Table 2: Study Design**

| **Group** | **N** | **Treatment** | **Dose (mg/kg)** | **Dosing Route** | **Schedule** |
|---|---|---|---|---|---|
| 1 | 10 | Vehicle 1+Vehicle 2 | -- | *p.o.* + *p.o.* | Qd x 35 (A.M.) + Qd x 35 (P.M.) |
| 2 | 10 | Niraparib | 31.4 mg/kg and adjusted to 50 mg/kg from PG-D5 | *p*.*o*. | Qd x 35 (P.M.) |
| 3 | 10 | Abiraterone | 200mg/kg | p.o.. | Qd x 35 (A.M.) |
| 4 | 10 | Abiraterone + Niraparib | 31.4 mg/kg and adjusted to 50 mg/kg from PG-D5 + 200mg/kg | *p.o.* + *p.o.* | Qd x 35 (A.M.) + Qd x 35 (P.M.) |

| | | | | | |
|---|---|---|---|---|---|
| Note: 1. N: number of animals; 2. Dosing volume: adjusted based on body weight (10 µL/g). 3. Dosed abiraterone first, followed by niraparib 7-8 hours later. | | | | | |

### Data analysis and statistics

Prism (GraphPad version 7) was used to graph all data and perform certain calculations, including median life span, survival and tumor volume. The log-rank Mantel-Cox test was used to evaluate survival data.

Tumor growth inhibition (TGI) was calculated as follows: TGI (%)=100 × (1-T/C). T and C are the mean tumor volume of the treated and control groups, respectively, on a given day. A p value of <0.05 was considered significant.

### Results

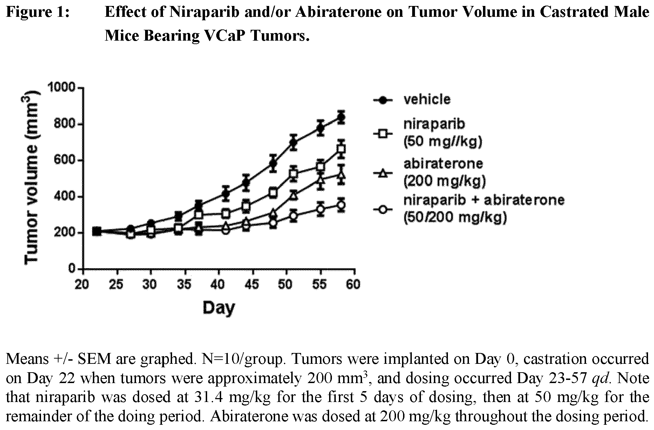

Each treatment inhibited tumor growth when compared with the vehicle control, during the dosing period of Day 23-57 (Figure 1). Niraparib inhibited tumor growth by approximately 25% from days 41-55, when compared with vehicle control. Abiraterone showed a greater inhibition of tumor growth from days 37-55 (ranging 35%-46%), when compared with the control group. The combination of niraparib and abiraterone showed the most dramatic inhibition of tumor growth from days 37-55 (ranging 38%-58%), when compared with the vehicle control group.

Tumor growth inhibition (TGI) on day 58 is calculated in Table 3 and shows that each treatment significantly reduced tumor growth compared with the control group. These results show that niraparib or abiraterone as single agents inhibited growth of the VCaP tumors, but that the combination of niraparib/abiraterone was most effective to inhibit tumor growth.

**Table 3. Antitumor Activity of the Test Compounds by Tumor Growth Inhibition Analysis**

| **Group** | **Treatment** | **Dose (mg/kg)** | **Tumor on day** | **TGI (%)b** | **p value^{c}** |
|---|---|---|---|---|---|
| 1 | Vehicle 1+ Vehicle 2 | - | 839±32 | - | -- |
| 2 | Niraparib | 31.4 mg/kg and adjusted to 50 mg/kg from PG-D5 mg/kg | 664±50 | 20.8 | 0.032 |
| 3 | Abiraterone | 200 mg/kg | 523±51 | 37.6 | <0.001 |
| 4 | Abiraterone + Niraparib | 200 mg/kg + 31.4 mg/kg and adjusted to 50 mg/kg from PG-D5 | 355±35 | 57.7 | <0.001 |

| | | | | | |
|---|---|---|---|---|---|
| Note: a: Mean ± SEM; b, c: vs. vehicle control. TGI, tumor growth inhibition. | | | | | |

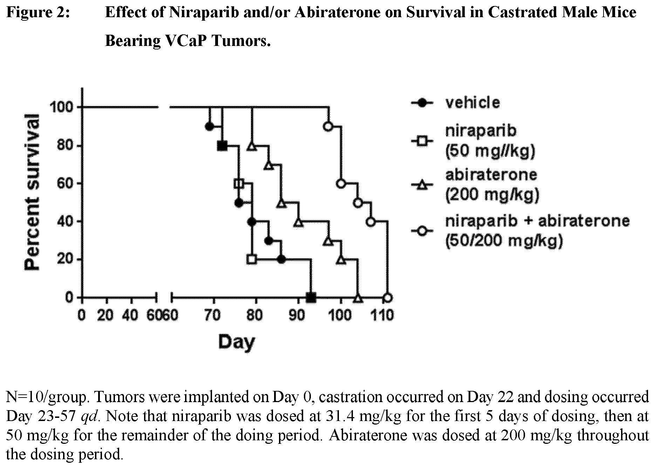

Survival curves and statistics are shown in Figure 2 and Table 4. The vehicle control group showed MLS of 77.5 days (Table 3). Niraparib alone did not increase MLS or provide a significant survival benefit when compared with the vehicle control group. Abiraterone alone increased the MLS by 10.5 days and significantly increased survival vs the vehicle control group (p = 0.0192). The combination niraparib/abiraterone group showed the greatest impact on survival, with an MLS of 105.5 days and significantly increased survival vs the vehicle control group (p < 0.0001). The niraparib/abiraterone treatment also significantly prolonged survival over the single agents niraparib (p < 0.0001) and abiraterone (p = 0.0021).

These results demonstrate that niraparib or abiraterone as single agents significantly prolonged the survival of mice bearing VCaP tumors, but that the combination of niraparib/abiraterone was most effective to prolong survival.

**Table 4. Effect of Niraparib and/or Abiraterone on the Survival of Tumor Bearing Mice**

| **Group** | **Treatment** | **MLS (days)^{a}** | **ILS (%)** | **P Value^{b}** |
|---|---|---|---|---|
| 1 | Vehicle 1+ Vehicle 2 | 77.5 (69-93) | - | - |
| 2 | Niraparib (31.4 mg/kg and adjusted to 50 mg/kg from PG-D5) | 79 (72-93) | -0.6 | 0.999 |
| 3 | Abiraterone (200 mg/kg) | 88 (79-104) | 13.1 | 0.026 |
| 4 | Abiraterone + Niraparib (200mg/kg + 31.4 mg/kg and adjusted to 50 mg/kg from PG-D5) | 105.5 (97-111) | 31.0 | <0.001 |

| | | | | |
|---|---|---|---|---|
| Note: a: MLS, median lifespan (survival ranges). b: The logrank test was used to determine the p values of survival between each treatment group compared with the vehicle control group. ILS, increased life span; compared with group 1. | | | | |

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

The following numbered embodiments are also presented.
1. A method for treating a prostate cancer comprising administering to a patient in need thereof a therapeutically effective amount of niraparib, a therapeutically effective amount of abiraterone acetate, and a therapeutically effective amount of prednisone.
2. The method according to embodiment 1, wherein the prostate cancer is hormone-sensitive or castration-resistant prostate cancer.
3. The method according to embodiment 2, wherein niraparib and abiraterone acetate, are administered to the patient in a single composition.
4. The method according to embodiment 2, wherein niraparib is administered in a separate composition from abiraterone acetate.
5. The method according to embodiment 1, wherein niraparib and abiraterone acetate are each administered once daily to the patient.
6. The method according to any one of embodiments 1, wherein niraparib is administered in a separate composition from the abiraterone acetate and prednisone.
7. The method according to any one of embodiments 1, wherein niraparib, abiraterone acetate, and prednisone are each administered in separate compositions.
8. The method according to embodiment 2, comprising administering about 500 to about 1500 mg/day of the abiraterone acetate to the patient.
9. The method according to embodiment 8 comprising administering about 600 to about 1300 mg/day of the abiraterone acetate to the patient.
10. The method according to embodiment 9 comprising administering about 900 to about 1100 mg/day of the abiraterone acetate to the patient.
11. The method according to embodiment 10, comprising administering about 1000 mg/day of the abiraterone acetate to the patient.
12. The method according to embodiment 11, comprising administering about 500 mg/day of the abiraterone acetate to the patient.
13. The method according to embodiment 8, comprising administering about 30 to about 400 mg/day of the niraparib to the patient.
14. The method according to embodiment 13, comprising administering about 100 to about 300 mg/day of the niraparib to the patient.
15. The method according embodiment 14, comprising administering about 175 to about 225 mg/day of the niraparib to the patient.
16. The method according to embodiment 11, comprising administering about 200 mg/day of the niraparib to the patient.
17. The method according to embodiment 12, comprising administering about 200 mg/day of the niraparib to the patient.
18. The method according to embodiment 13, comprising administering about 1 to about 25 mg/day of the prednisone to the patient.
19. The method according to embodiment 14, comprising administering about 5 to about 15 mg/day of the prednisone to the patient.
20. The method according to embodiment 15, comprising administering about 9 to about 11 mg/day of the prednisone to the patient.
21. The method according to embodiment 16, comprising administering about 10 mg/day of the prednisone to the patient.
22. The method according to embodiment 17, comprising administering about 10 mg/day of the prednisone to the patient.
23. The method according to embodiment 16, comprising administering about 5 mg/day of the prednisone to the patient.
24. The method according to embodiment 17, comprising administering about 5 mg/day of the prednisone to the patient.
25. The method according to embodiment 1, comprising administering the niraparib and the abiraterone acetate to the patient once per day over multiple days, and increasing the dosage of one or both of the niraparib and abiraterone acetate at least once over time.
26. The method according to embodiment 5 wherein the dosing of niraparib is increased at least once over time.
27. The method according to embodiment 25, comprising administering the prednisone to the patient twice per day over multiple days, and increasing the dosage of the prednisone at least once over time.
28. The method according to embodiment 1, comprising orally administering the niraparib, the abiraterone acetate, and the prednisone to the patient.
29. The method according to any preceding embodiment 1, wherein the patient underwent taxane-based chemotherapy prior to administering a first dose of the niraparib and the abiraterone acetate.
30. The method according to embodiment 1, wherein the patient underwent at least one line of androgen receptor-targeted therapy prior to administering a first dose of the niraparib and the abiraterone acetate
31. A pharmaceutical composition comprising niraparib, abiraterone acetate, and prednison in a total amount that is in a single oral dosage form therapeutically effective for the treatment of prostate cancer in a human patient.
32. The composition according to embodiment 1, wherein said composition is configured for oral administration.
33. The composition according to embodiment 31 or embodiment 3232 wherein the composition comprises about 100 to about 350 mg of the niraparib, about 100 to about 1500 mg of the abiraterone acetate, and about 2 to about 15 mg of the prednisone.
34. A pharmaceutical composition comprising niraparib, abiraterone acetate, and prednisone in a total amount that is in multiple oral dosage forms therapeutically effective for the treatment of prostate cancer in a human patient.
35. The composition according to embodiment 33, wherein said composition is configured for oral administration.
36. The composition according to embodiment 34 or embodiment 35, wherein the composition comprises about 100 to about 350 mg of the niraparib, about 100 to about 1500 mg of the abiraterone acetate, and about 2 to about 15 mg of the prednisone.

## Claims

1. Niraparib and abiraterone acetate for use in a method for treating a prostate cancer comprising administering to a patient in need thereof a therapeutically effective amount of niraparib, and a therapeutically effective amount of abiraterone acetate.

2. The niraparib and abiraterone acetate for use according to claim 1, wherein the niraparib and abiraterone acetate are administered or combined with a steroid, such as a corticosteroid or a glucocorticoid.

3. The niraparib and abiraterone acetate for use according to claim 2, wherein the steroid is prednisone or prednisolone.

4. The niraparib and abiraterone acetate for use according to any preceding claim, wherein the prostate cancer is metastatic castration-resistant prostate cancer (mCRPC), hormone-sensitive prostate cancer, or castration-resistant prostate cancer.

5. The niraparib and abiraterone acetate for use according to any preceding claim, wherein niraparib is administered in a separate composition from abiraterone acetate.

6. The niraparib and abiraterone acetate for use according to any preceding claim, wherein niraparib is administered in a separate composition from abiraterone acetate, wherein niraparib is administered in a separate composition from the abiraterone acetate and steroid, wherein the steroid is administered in a separate composition to the niraparib and abiraterone acetate, or wherein niraparib, abiraterone acetate, and steroid are each administered in separate compositions.

7. The niraparib and abiraterone acetate for use according to any one of claims 1 to 4 wherein niraparib and abiraterone acetate are combined in a single composition, preferably a single pill.

8. The niraparib and abiraterone acetate for use according to any preceding claim, wherein niraparib and abiraterone acetate are each administered once daily to the patient.

9. The niraparib and abiraterone acetate for use according to any preceding claim, comprising administering about 500 to about 1500 mg/day, about 600 to about 1300 mg/day, or about 900 to about 1100 mg/day of the abiraterone acetate to the patient.

10. The niraparib and abiraterone acetate for use according to claim 9, comprising administering about 1000 mg/day or about 500 mg/day of the abiraterone acetate to the patient.

11. The niraparib and abiraterone acetate for use according to any preceding claim , comprising administering about 30 to about 400 mg/day, about 100 to about 300 mg/day, or about 175 to about 225 mg/day of the niraparib to the patient.

12. The niraparib and abiraterone acetate for use according to claim 11, comprising administering about 200 mg/day of the niraparib to the patient.

13. The niraparib and abiraterone acetate for use according to any preceding claim wherein the patient is positive for homologous recombination repair gene alterations.

14. The niraparib and abiraterone acetate for use according to claim 13 wherein the gene alterations are BRCA (BRCA2 and/or BRCA1) gene alterations.

15. The niraparib and abiraterone acetate for use according to claim 13 wherein the gene alterations are BRCA2, BRCA1, PALB2, and/or CHEK2 gene alterations.

16. The niraparib and abiraterone acetate for use according to claim 13 wherein the gene alterations are BRCA2, BRCA1, CHEK2, HDAC2, BRIP1, FANCA, and/or PALB2 gene alterations.

17. The niraparib and abiraterone acetate for use according to any preceding claim, comprising administering about 1 to about 25 mg/day, about 5 to about 15 mg/day, or about 9 to about 11 mg/day of steroid to the patient.

18. The niraparib and abiraterone acetate for use according to claim 17, comprising administering about 10 mg/day or about 5 mg/day of steroid to the patient.

19. The niraparib and abiraterone acetate for use according to any preceding claim, comprising orally administering the niraparib, the abiraterone acetate, and optionally the steroid to the patient.

20. The niraparib and abiraterone acetate for use according to any preceding claim, wherein the patient underwent taxane-based chemotherapy prior to administering a first dose of the niraparib and the abiraterone acetate, and optionally the steroid; and/or wherein the patient underwent at least one line of androgen receptor-targeted therapy prior to administering a first dose of the niraparib and the abiraterone acetate, and optionally the steroid.

21. A pharmaceutical composition comprising niraparib and abiraterone acetate in a single composition, preferably a single pill, in a total amount that is therapeutically effective for the treatment of prostate cancer in a human patient.

22. The composition according to claim 20, wherein said composition is configured for oral administration, and/or wherein the composition comprises about 100 to about 350 mg of the niraparib, and about 100 to about 1500 mg of the abiraterone acetate.

23. The niraparib and abiraterone acetate for use according to any of claims 1-20, or the composition according to claim 21 or 22, wherein the niraparib is niraparib tosylate.

24. The niraparib and abiraterone acetate for use according to any of claims 1-20, or the composition according to claim 21 or 22, wherein the niraparib is (3S)-3-{ 4-[7-(aminocarbonyl)-2H-indazol-2-yl]phenyl}piperidinium 4-methylbenzenesulfonate monohydrate.
